# EUROPEAN PATENT APPLICATION

(11) **EP 4 424 671 A1**
(43) Date of publication of application: **04.09.2024**
(21) Application number: 22887535.7
(22) Date of filing: 24.10.2022
(51) Int. Cl.: C07D 217/02, A61K 31/472, A61K 31/4725, A61K 31/4985, A61K 31/517, A61K 31/4375, A61K 31/437, A23L 33/10, C07D 405/04, C07D 409/04

(54) **NOVEL TRICYCLIC DERIVATIVE COMPOUND AND USE THEREOF**

(30) Priority: 27.10.2021 KR 20210144666; 21.10.2022 KR 20220136853
(71) Applicant: Huchembio Co., Ltd., Hwaseong-si, Gyeonggi-do 18469 (KR)
(72) Inventor: LEE, Sang Hak, Hwaseong-si Gyeonggi-do 18497 (KR); YOON, Mi Ja, Hwaseong-si Gyeonggi-do 18497 (KR); KIM, Mi Sun, Gwacheon-si Gyeonggi-do 13835 (KR); LEE, Do Hyung, Suwon-si Gyeonggi-do 16287 (KR); KANG, Seung Hee, Bucheon-si Gyeonggi-do 14574 (KR)
(74) Representative: Jacobi, Markus Alexander
(86) International application number: PCT/KR2022/016290
(87) International publication number: WO 2023/075339

(57) **Abstract**

The present invention relates to a novel tricyclic derivative compound or a pharmaceutically acceptable salt thereof and a use thereof, in which it has excellent STAT3 inhibitory activity and was found to exhibit an excellent inhibitory effect compared to known compounds in phase-3 clinical trials, and thus can be used as a therapeutic agent for STAT3 related diseases.

## Description

### [Technical Field]

The present invention relates to a novel tricyclic derivative compound and a use thereof.

### [Background Art]

STAT is an STAT protein first discovered in 1994 and is known to play a major role in cytokine and growth factor signaling pathways. The STAT family consists of seven structurally and functionally similar proteins, including STAT1, STAT2, STAT3, STAT4, STAT5A, STAT5B and STAT6, which have been shown to regulate cell proliferation, survival, differentiation, angiogenesis, and related gene expression.

STAT is an inactive transcription factor localized to the cytoplasm. Binding of many cytokines, hormones, and growth factors to a corresponding receptor activates a receptor-associated Janus-activated kinase (JAK) which may phosphorylate tyrosine residues in the cytoplasmic domain of the receptor, thus providing a docking site in the SRC homology 2 (SH2) domain. As a result, these STAT proteins are activated by JAK through phosphorylation of tyrosine residues at C-terminus, thus leading to homodimerization or heterodimerization and activation. Finally, the STAT dimer migrates to a nucleus and binds to a specific sequence in a promoter of a target gene to induce transcription.

In particular, the transcriptional regulator STAT3 plays an important role in vertebrate development and mature tissue functions, including inflammatory and immune regulation. Mutations in human STAT3 are associated with immunodeficiency, autoimmunity and diseases such as cancer. Surprisingly, however, hyperactivation or inactivation of STAT3 leads to human diseases, thus indicating that strictly regulated STAT3 function is at the heart of health. Abnormal STAT3 function is used to control a mechanism leading to human diseases and disease outcomes, and in particular, activated STAT-3 may down-regulate lymphocyte formation, E-cadherin expression by vascular endothelial cells, Treg cell activity, and antiviral immune responses (including type I IFN-mediated signals, NK cell activity, Th1 cell response, and CD8+CTL). Activated STAT-3 may up-regulate lymphopenia, vascular leakage, hyperinflammation and viral persistence, formation of extracellular matrix, expression of plasminogen activator inhibitor-1, polarization and activation of Th17 cells, and polarization of M2 macrophages which promote pulmonary fibrosis, thrombosis, hyperinflammation/cytokine storm. Referring to the above-mentioned various papers or literature announcements, the development of STAT-3 inhibitors enables the development of excellent drugs capable of treating diseases related to inflammation and autoimmunity.

### [Disclosure]

### [Technical Problem]

An object of the present invention is to provide a novel tricyclic derivative compound or a pharmaceutically acceptable salt thereof.

Another object of the present invention is to provide a pharmaceutical composition for preventing or treating STAT3-associated diseases, including the compound or the pharmaceutically acceptable salt thereof as an active ingredient.

Another object of the present invention is to provide a health functional food composition for preventing or ameliorating STAT3-associated diseases, including the compound 1 or the pharmaceutically acceptable salt thereof as an active ingredient.

Another object of the present invention is to provide a reagent composition for inhibiting STAT3 activity, including the compound or the pharmaceutically acceptable salt thereof as an active ingredient.

### [Technical Solution]

To achieve the above objects, the present invention may provide a tricyclic derivative compound represented by formula 1 below or a pharmaceutically acceptable salt thereof:

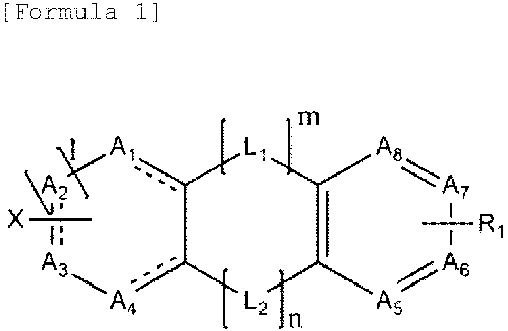

in which in above formula 1, A₁ to A₈ may be the same as or different from each other and may be CH or N, L₁ and L₂ may be the same as or different from each other and may be any one of CH₂, CO, NH, O, S, SO, SO₂, NR₂, and CHR₂, l and n may be the same as or different from each other and may be 0 or 1, m may be 1 or 2, X may be any one of H, halogen, (C1-C4) alkyl, and (C1-C4) alkoxy, R₁ may be any one of 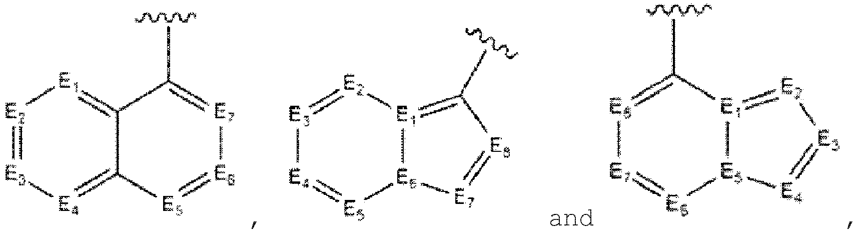 E₁ to E₈ may be the same as or different from each other and may be CH or N, and R₂ may be selected from the group consisting of H, a hydroxy group, an ether group, halogen, a carbonyl group, a nitro group, a naphthyl group, a cyano group, an amino group, an amidino group, hydrazine, hydrazone, a carboxyl group, a sulfonic acid group, a phosphoric acid group, (C1-C12)alkyl, (C1-C12)alkenyl, (C1-C12)alkynyl, (C6-C10)aryl, and (C7-C10) arylalkyl.

In addition, the present invention may provide a pharmaceutical composition for preventing or treating STAT3-associated diseases, including the compound or the pharmaceutically acceptable salt thereof as an active ingredient.

Furthermore, the present invention may provide a health functional food composition for preventing or ameliorating STAT3-associated diseases, including the compound or the pharmaceutically acceptable salt thereof as an active ingredient.

Moreover, the present invention may provide a reagent composition for inhibiting STAT3 transcriptional activity, including the compound or the pharmaceutically acceptable salt thereof as an active ingredient.

### [Advantageous Effects]

The present invention relates to a novel tricyclic derivative compound or a pharmaceutically acceptable salt thereof and a use thereof. It has been confirmed that the compound has a more excellent STAT3 transcriptional activity inhibitory effect than that of BBI608 which is under an ongoing phase III clinical trial and has an excellent STAT3 inhibitory activity which has not been known to date, and thus can be used as a therapeutic agent for STAT3-associated diseases.

### [Description of Drawings]

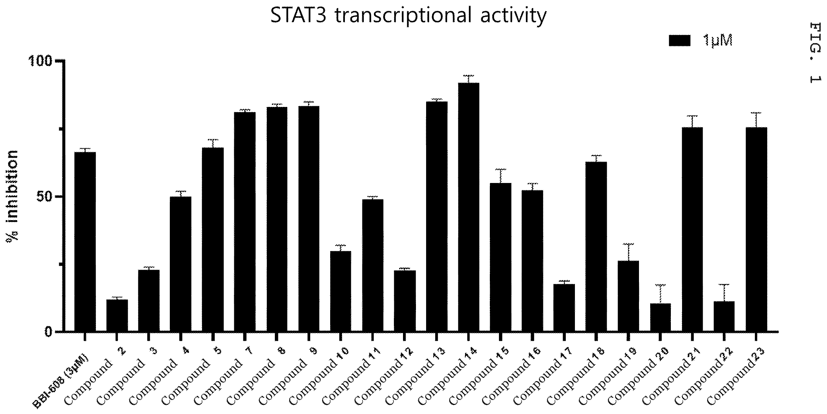

FIG. 1 is a graph showing the results of an experiment on a STAT3 inhibitory effect of novel tricyclic derivative compounds.

### Best Mode

Throughout the specification of the present invention, when any part is said to "include" a certain component, this means that the part may further include other components rather than excluding the other components, unless otherwise particularly specified.

Hereinafter, the present invention will be described in more detail.

The present invention may provide a tricyclic derivative compound represented by formula 1 below or a pharmaceutically acceptable salt thereof:

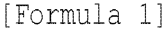

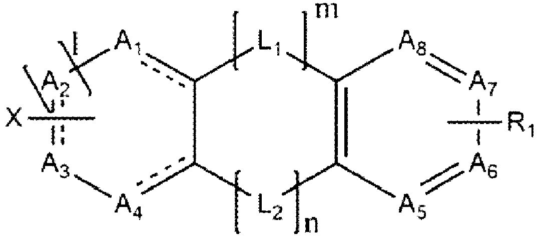

in which in above formula 1, A₁ to A₈ may be the same as or different from each other and may be CH or N, L₁ and L₂ may be the same as or different from each other and may be any one of CH₂, CO, NH, O, S, SO, SO₂, NR₂ and CHR₂, l and n may be the same as or different from each other and may be 0 or 1, m may be 1 or 2, and X may be any one of H, halogen, (C1-C4) alkyl, and (C1-C4) alkoxy; and R₁ may be any one of 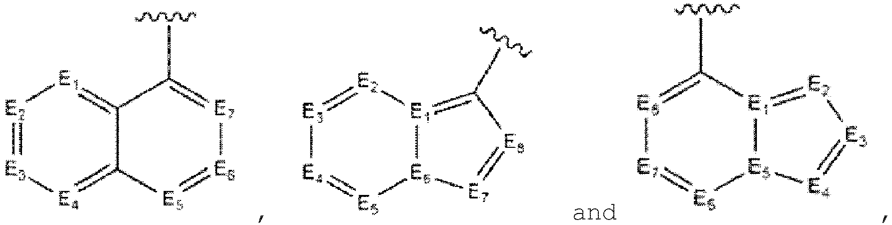 E₁ to E₈ may be the same as or different from each other and may be CH or N, and R₂ may be selected from the group consisting of H, a hydroxy group, an ether group, halogen, a carbonyl group, a nitro group, a naphthyl group, a cyano group, an amino group, an amidino group, hydrazine, hydrazone, a carboxyl group, a sulfonic acid group, a phosphoric acid group, (C1-C12)alkyl, (C1-C12)alkenyl, (C1-C12)alkynyl, (C6-C10)aryl, and (C7-C10)arylalkyl.

Above formula 1 may be formula 1-1 below: 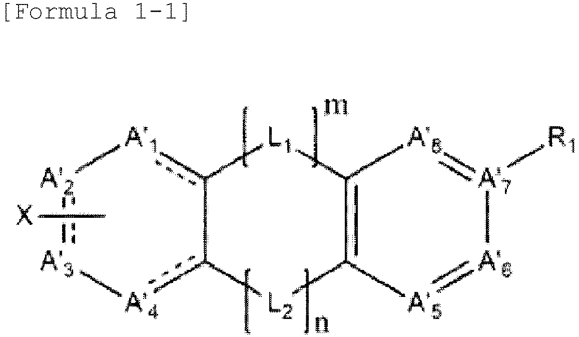 in which in above formula 1-1, A'₁ to A'₈ may be the same as or different from each other and may be CH or N, L₁ and L₂ may be the same as or different from each other and may be any one of CH₂, CO, NH, O, S, and SO, n may be 0 or 1, m may be 1 or 2, and X may be H or halogen; and R₁ may be any one of 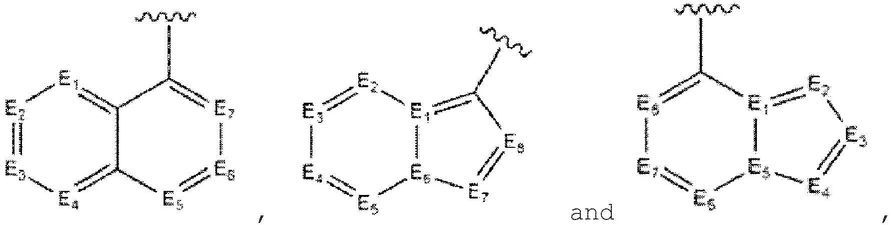 and E₁ to E₈ may be the same as or different from each other and may be CH or N.

In above formula 1-1, A'₁, A'₂, and A'₄ to A'₈ may be CH, A'₃ may be CH or N, L₁ and L₂ may be the same as or different from each other and may be any one of CO, NH, O and S, and X may be H or Br.

The compound may be selected from the group consisting of 3-(isoquinolin-4-yl)-9H-fluoren-9-one, 2-bromo-7-(isoquinolin-4-yl)phenanthren-9,10-dione, 2-(isoquinolin-4-yl)-9H-xanthen-9-one, 2-(isoquinolin-4-yl)-9H-thioxanthen-9-one, 2-(pyrido[3,4-b]pyrazin-8-yl)-9H-fluoren-9-one, 2-(quinazolin-4-yl)-9H-fluoren-9-one, 2-(1,6-naphthyridin-8-yl)-9H-fluoren-9-one, 2-(1,6-naphthyridin-5-yl)-9H-fluoren-9-one, 2-(pyrazolo[1,5-a]pyridin-3-yl)-9H-fluoren-9-one, 2-(imidazo[1,2-a]pyridin-3-yl)-9H-fluoren-9-one, 4-(dibenzo[b,d]thiophen-3-yl)isoquinoline, 4-(dibenzo[b,d]furan-3-yl)isoquinoline, 7-(isoquinolin-4-yl)-5H-pyrido[4,3-b]indole, 2-(isoquinolin-4-yl)-9H-fluoren-9-one, 2-(isoquinolin-4-yl)-9H-carbazole, 7-(isoquinolin-4-yl)-2,3,4,9-tetrahydro-1H-carbazole, 2-(isoquinolin-8-yl)-9H-fluoren-9-one, 2-(isoquinolin-5-yl)-9H-fluoren-9-one, 2-(imidazo[1,2-a]pyridin-5-yl)-9H-fluoren-9-one, 2-([1,2,4]triazolo[4,3-a]pyridin-8-yl)-9H-fluoren-9-one, 2-(isoquinolin-1-yl)-9H-fluoren-9-one, and 2-(imidazo[1,2-a]pyridin-8-yl)-9H-fluoren-9-one.

Above formula 1 may be formula 1-2 below: 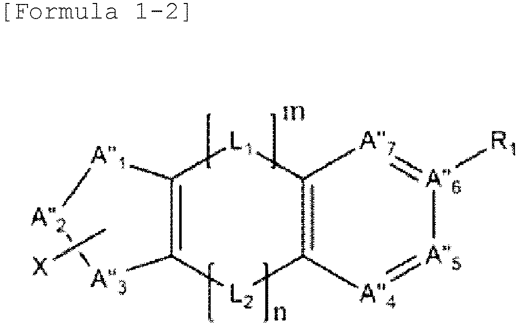 in which in above formula 1-1, A"₁ to A"₇ may be the same as or different from each other and may be CH or N, L₁ and L₂ may be the same as or different from and may be any one of CH₂, CO, NH, O, S, and SO, n may be 0 or 1, m may be 1 or 2, and X may be H or halogen; and R₁ may be any one of 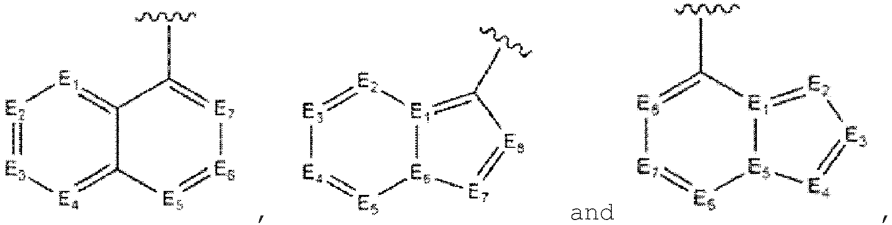 and E₁ to E₈ may be the same as or different from each other and may be CH or N.

In above formula 1-2, A"₁ to A"₇ may be CH, n may be 0, m may be 1, L₁ may be CH₂ or NH, and X may be H.

The compound may be 6-(isoquinolin-4-yl)-1,2,3,4-tetrahydrocyclopenta[b]indole.

In addition, the present invention may provide a pharmaceutical composition for preventing or treating STAT3-associated diseases, including the compound or the pharmaceutically acceptable salt thereof as an active ingredient.

The STAT3-associated diseases may be any one of cancer, osteoporosis, atopy, viral inflammatory disease, diabetic retinopathy, diabetes, hemophilic arthropathy, atherosclerosis, keloid, wound granulation, vascular adhesion, autoimmune disease, restenosis, intestinal tract adhesion, cat scratch disease, ulcer, cirrhosis, diabetic nephropathy, malignant nephrosclerosis, thrombotic microangiopathy, organ transplant rejection, glomerulopathy, neurodegenerative disease, and inflammatory disease.

In another embodiment of the present invention, the pharmaceutical composition may further include at least one additive selected from the group consisting of suitable carriers, excipients, disintegrants, sweeteners, coating agents, expanding agents, lubricants, glidants, flavoring agents, antioxidants, buffers, bacteriostats, diluents, dispersing agents, surfactants, binders, and lubricants commonly used in the preparation of pharmaceutical compositions.

Specifically, the carrier, excipient, and diluent used may include lactose, dextrose, sucrose, sorbitol, mannitol, xylitol, erythritol, maltitol, starch, acacia rubber, alginate, gelatin, calcium phosphate, calcium silicate, cellulose, methyl cellulose, microcrystalline cellulose, polyvinyl pyrrolidone, water, methyl hydroxybenzoate, propyl hydroxybenzoate, talc, magnesium stearate, and mineral oil, and a solid formulation for oral administration may include tablets, pills, powders, granules, capsules, and the like, and the solid formulation may be prepared by mixing the composition with at least one excipient, for example, starch, calcium carbonate, sucrose, lactose, gelatin, or the like. In addition, lubricants such as magnesium stearate and talc may be also used in addition to simple excipients. A liquid formulation for oral administration may include suspensions, liquids for internal use, emulsions, syrups, etc., but may also include various excipients, for example, humectants, sweetening agents, flavoring agents, preservatives, etc. in addition to water and liquid paraffin, which are the frequently used simple diluents. A formulation for parenteral administration may include sterile aqueous solutions, non-aqueous solvents, suspensions, emulsions, lyophilized preparations, suppositories, etc. The non-aqueous solvent and the suspending agent used may include propylene glycol, polyethylene glycol, vegetable oil like olive oil, injectable ester like ethyl oleate, etc. A base of the suppository used may include witepsol, macrogol, tween 61, cacao butter, laurinum, glycerogelatin, etc.

According to one embodiment of the present invention, the pharmaceutical composition may be administered to a subject in a conventional manner via an intravenous, intraarterial, intraperitoneal, intramuscular, intraarterial, intrasternal, percutaneous, nasal, inhalation, topical, rectal, oral, intraocular, or intradermal route.

The dosage of the active ingredient according to the present invention may vary depending on the condition and weight of the subject, the type and degree of disease, a drug form, and an administration route and period, and may be appropriately selected by those skilled in the art, and a daily dosage may be 0.01 mg/kg to 200 mg/kg, preferably 0.1 mg/kg to 200 mg/kg, and more preferably 0.1 mg/kg to 100 mg/kg. The administration may be performed once a day or divided into several doses, by which the scope of the present invention is not limited.

Furthermore, the present invention may provide a health functional food composition for preventing or ameliorating STAT3-associated diseases, including the compound or the pharmaceutically acceptable salt thereof as an active ingredient.

The health functional food may contain various nutrients, vitamins, minerals (electrolytes), flavoring agents such as synthetic flavoring agents, natural flavoring agents and the like, coloring agents and enhancers (cheese, chocolate, etc.), pectic acid and salts thereof, alginic acid and salts thereof, organic acid, protective colloidal thickeners, pH adjusting agents, stabilizers, preservatives, glycerin, alcohol, carbonators used in carbonated beverages, etc.

Besides, it may also contain flesh for preparing natural fruit juices, synthetic fruit juices and vegetable beverages. Such ingredients may be used independently or in combination. In addition, the health functional food composition may be in the form of any one of meat, sausage, bread, chocolate, candies, snacks, confectionery, pizza, ramen, gum, ice cream, soup, beverage, tea, functional water, drinks, alcohol, and vitamin complex.

In addition, the health functional food may further include a food additive, and the suitability as a "food additive" may be determined according to the specifications and standards for the relevant item in accordance with the general rules, general test methods and the like of the Food Additive Code approved by the Ministry of Food and Drug Safety, unless otherwise specified.

As items listed on said "Food Additives Code," there may be, for example, chemical compounds such as ketones, glycine, potassium citrate, nicotinic acid, cinnamic acid, etc.; natural additives such as persimmon color, licorice extract, crystalline cellulose, kaoliang color, guar gum, etc.; and mixed formulations such as L-sodium glutamate formulation, alkali additives for noodles, preservatives formulation, tar color formulation, etc.

In this case, in the process of preparing the health functional food, the content of the active ingredient to be added to the food may be appropriately increased or decreased as necessary, and preferably, the active ingredient may be added in an amount of 1 part by weight to 90 parts by weight based on 100 parts by weight of the food.

In addition, the present invention may provide a reagent composition for inhibiting STAT3 activity, including the compound or the pharmaceutically acceptable salt thereof as an active ingredient.

### [Mode for Invention]

Hereinafter, the present invention will be described with reference to examples and experimental examples. However, the following examples and experimental examples are provided only for the purpose of illustrating the present invention, and thus the content of the present invention is not limited thereto.

### [Synthesis Example] Synthesis of tricyclic derivative compound

A synthesis reaction for all compounds of the present invention was carried out according to Suzuki*Miyaura reaction. The synthesis method may be as follows.

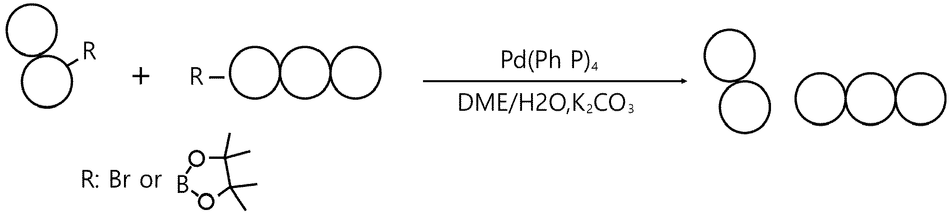

A bromide compound (1 eq) and a boronic pinacolester compound (1 eq) were dissolved in DME/H₂O 4:1 and stirred. Then, K₂CO₃ (3 eq) and Pd(Ph₃P)₄ (0.2 eq) were added thereto, and the resulting mixture was heated and stirred at 80°C. After stirring for three hours, the reaction was identified by TLC, and when the reaction was completed, the temperature was lowered to room temperature. Salt water and ethyl acetate were added to perform extraction, and then washed twice with salt water. Na₂SO₄ was added to the extracted ethyl acetate, dried, and then filtered. The filtered solution was dried under reduced pressure and purified through a column to obtain a target compound.

Through the synthesis, the present inventors synthesized the following compounds.

### <Compound 1>

### 3-(isoquinolin-4-yl)-9H-fluoren-9-one

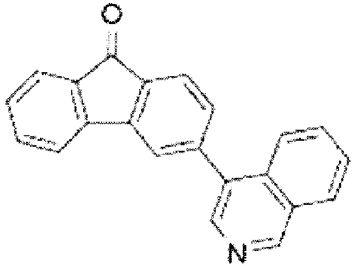

NMR(H): 9.45(s,1H), 8.59(s,1H), 8.29(d,1H), 8.07(s,1H), 8.07~7.85(m,3H), 7.80(d,2H), 7.65(dd,2H), 7.57(d,1H), 7.40(t,1H); MS:308.48

### <Compound 2>

### 2-bromo-7-(isoquinolin-4-yl)phenanthrene-9,10-dione

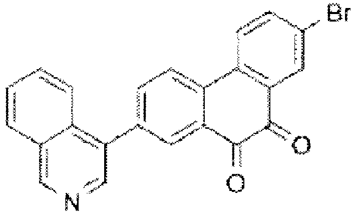

H NMR: 9.45(s,1H), 8.56(s,1H), 8.53(d,1H), 8.38(d,1H), 8.28(d,1H), 8.15(d,2H), 8.02~7.95(m,2H), 7.87(s,2H), 7.82~7.78(m,1H); MS:413.95

### <Compound 3>

### 2-(isoquinolin-4-yl)-9H-xanthen-9-one

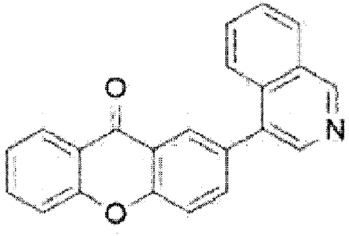

H NMR: 9.42(s,1H), 8.55(s,1H), 8.28~8.23(m,3H), 8.08(d,1H), 7.98~7.75(m,6H), 7.55(t,1H); MS:324.50

### <Compound 4>

### 2-(isoquinolin-4-yl)-9H-thioxanthen-9-one

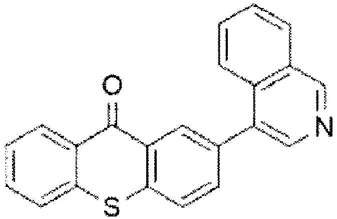

H NMR: 9.43(s,1H), 8.6~8.55(m,2H) 8.51(d,1H), 8.28(d,1H), 8.10(d,1H), 8.02(d,1H), 7.93(d,1H), 7.88~7.75(m,4H), 7.65(t,1H); MS:340.35

### <Compound 5>

### 2-(pyrido[3,4-b]pyrazin-8-yl)-9H-fluoren-9-one

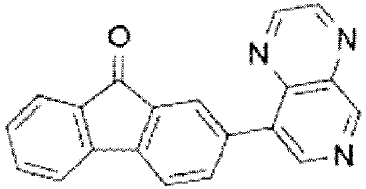

H NMR: 9.57(s,1H), 9.25(s,1H), 9.18(s,1H), 9.02(s,1H), 8.01(s,3H), 7.93(d,1H), 7.69~7.65(m,2H), 7.45(t,1H); MS:310.23

### <Compound 6>

### 2-(quinazolin-4-yl)-9H-fluoren-9-one

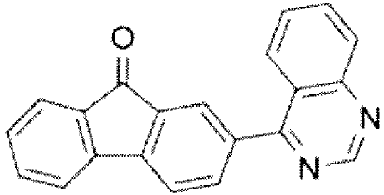

H NMR: 9.38(s,1H), 8.20~8.08(m,5H), 7.98~7.92(m,2H), 7.75(t,1H), 7.72~7.68(m,2H), 7.49(t,1H); MS:309.15

### <Compound 7>

### 2-(1,6-naphthyridin-8-yl)-9H-fluoren-9-one

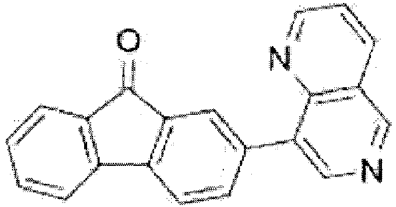

H NMR: 9.47(s,1H), 9.20(d,1H), 8.89(s,1H), 8.70(d,1H), 8.02~7.92(m,3H), 7.89(d,1H), 7.80(dd,1H), 7.70~7.62(m,2H), 7.45(t,1H); MS:309.24

### <Compound 8>

### 2-(1,6-naphthyridin-5-yl)-9H-fluoren-9-one

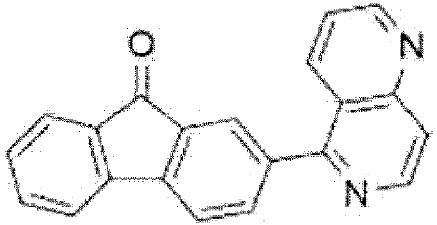

H NMR: 9.47 (s,1H), 9.14(d,1H), 8.70(s,1H), 8.33(d,1H), 8.03(d,1H), 7.93(d,1H), 7.84(d,2H), 7.76(s,1H), 7.70~7.62(m,2H) 7.45(t,1H); MS:309.29

### <Compound 9>

### 2-(pyrazolo[1,5-a]pyridin-3-yl)-9H-fluoren-9-one

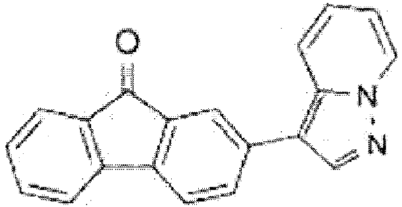

NMR (H) : 8.78 (d, 1H), 8.54 (S, 1H), 8.03 (d, 1H), 7.95 (d, 1H), 7.95~7.89(m,3H), 7.68~7.63(m, 2H), 7.40(q, 2H), 7.03(t, 1H); MS+:297.40

### <Compound 10>

### 2-(imidazo[1,2-a]pyridin-3-yl)-9H-fluoren-9-one

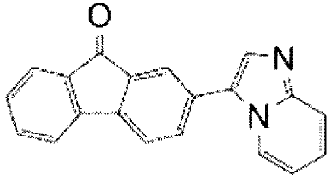

NMR (H) : 8.62 (d, 1H), 8.54 (S, 1H), 8.40 (d, 1H), 8.08 (1H, S), 7.95(d, 1H), 7.90(d, 1H), 7.70~7.65(m, 4H), 7.43(t, 1H), 7.03(t, 1H); MS+:297.54

### <Compound 11>

### 4-(dibenzo[b,d]thiophen-3-yl)isoquinoline

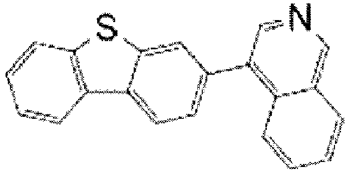

NMR(H): 9.40(s, 1H), 8.57(d, 2H), 8.49(d, 1H), 8.30(d, 2H), 8.10(d, 1H), 7.92(d, 1H), 7.83(t, 1H), 7.75(t, 1H), 7.70(d, 1H), 7.58(d, 2H); MS+:312.89

### <Compound 12>

### 4-(dibenzo[b,d]furan-3-yl)isoquinoline

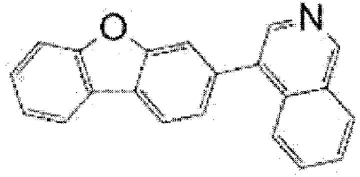

MS+: 296.37

### <Compound 13>

### 7-(isoquinolin-4-yl)-5H-pyrido[4,3-b]indole

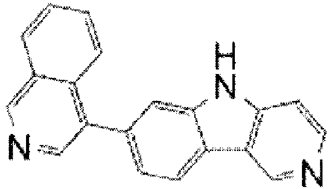

NMR(H): 9.39(d, 2H), 8.55(s, 1H), 8.43(d, 1H), 8.41(d, 1H), 8.25(d, 1H), 8.00(d, 1H), 7.82~7.74(m, 3H), 7.65(br, 1H), 7.40(d, 1H); MS+:296.28

### <Compound 14>

### 2-(isoquinolin-4-yl)-9H-fluoren-9-one

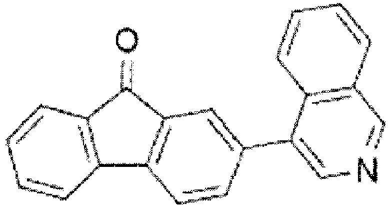

H NMR: 9.30(s,1H), 8.52(s,1H), 8.07(d,1H), 7.90(d,1H), 7.84(s,1H), 7.74~7.56(m,7H), 7.38(t,1H); MS:308.04

### <Compound 15>

### 2-(isoquinolin-4-yl)-9H-carbazole

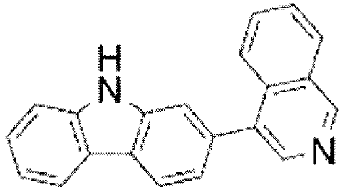

H NMR: 9.30(s,1H), 8.60(s,1H), 8.27(s,1H), 8.21(d,1H), 8.14(d,1H), 8.12~8.01(m,2H), 7.69~7.64(m,2H), 7.58(s,1H), 7.51(t,2H), 7.40(d,1H), 7.30~7.26(m,1H); MS+: 295.40

### <Compound 16>

### 7-(isoquinolin-4-yl)-2,3,4,9-tetrahydro-1H-carbazole

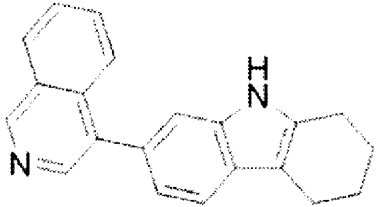

NMR (DMSO d6) : 10.82 (s, 1H), 9.29 (s, 1H), 8.45 (s, 1H), 8.20 (d, 1H), 7.95 (d, 1H), 7.76 (m, 1H), 7.71 (m, 1H), 7.50 (d, 1H), 7.39 (d, 1H), 7.09 (dd, 1H), 2.75 (m, 2H), 2.69 (m, 2H), 1.86 (m, 4H) ;
MS+ : 299.25

### <Compound 17>

### 2-(isoquinolin-8-yl)-9H-fluoren-9-one

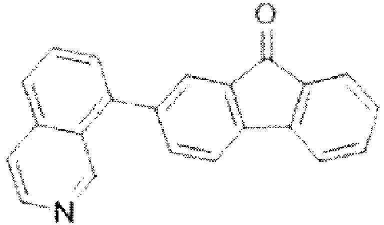

NMR (CDCl3 ): 9.33 (s, 1H), 8.58 (d, 1H), 7.90 (d, 1H), 7.83 (d, 1H), 7.79 (m, 2H), 7.73 (d, 1H), 7.70 (dd, 1H), 7.65 (dd, 1H), 7.63 (m, 1H), 7.60 (dd, 1H), 7.56 (m, 1H), 7.36 (m, 1H) ;
MS+ : 308.22

### <Compound 18>

### 2-(isoquinolin-5-yl)-9H-fluoren-9-one

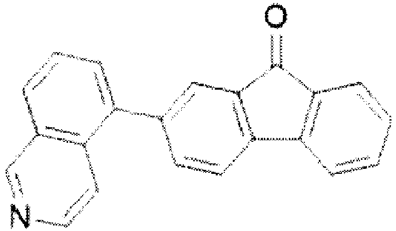

NMR (CDCl3 ): 9.36 (s, 1H), 8.52 (d, 1H), 8.07 (dd, 1H), 7.80 (d, 1H), 7.73 (m, 5H), 7.62 (d, 2H), 7.56 (m, 1H), 7.36 (m, 1H) ;
MS+ : 308.26

### <Compound 19>

### 2-(imidazo[1,2-a]pyridin-5-yl)-9H-fluoren-9-one

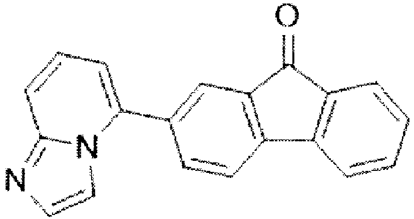

NMR (CDCl3 ): 7.94 (d. 1H), 7.79 (dd, 1H), 7.69 (m, 6H), 7.57 (m, 1H), 7.39 (m, 1H), 7.31 (m, 1H), 6.84 (d, 1H) ;
MS+ : 297.24

### <Compound 20>

### 2-([1,2,4]triazolo[4,3-a]pyridin-8-yl)-9H-fluoren-9-one

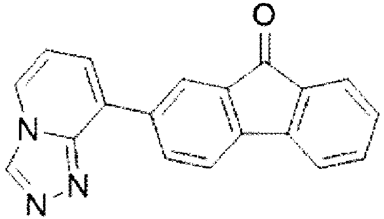

NMR (DMSO d6 ): 9.29 (d, 1H), 8.06 (s, 2H), 7.96 (m, 2H), 7.85 (d, 1H), 7.71 (m, 2H), 7.49 (m, 2H), 7.13 (dd, 1H) ;
MS+ : 298.23

### <Compound 21>

### 6-(isoquinolin-4-yl)-1,2,3,4-tetrahydrocyclopenta[b]indole

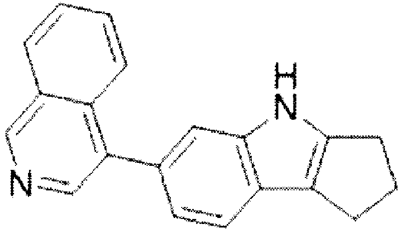

NMR (CDCl3 ): 9.25 (s, 1H), 8.54 (s, 1H), 8.05 (m, 3H), 7.65 (m, 2H), 7.57 (d, 1H), 7.47 (d, 1H), 7.23 (dd, 1H), 2.92 (m, 4H), 2.60 (m, 2H) ;
MS+ : 285.27

### <Compound 22>

### 2-(isoquinolin-1-yl)-9H-fluoren-9-one

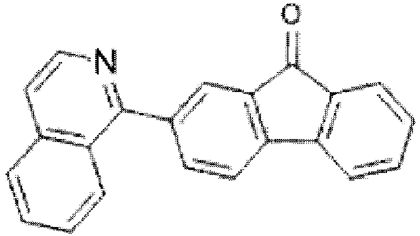

NMR(CDCl3) : 8.63(d,1H), 8.11(d,1H), 8.02 (s, 1H), 7.91(t,2H), 7.71(m,4H), 7.58(m,3H), 7.35(t,1H);
MS+:308.26

### <Compound 23>

### 2-(imidazo[1,2-a]pyridin-8-yl)-9H-fluoren-9-one

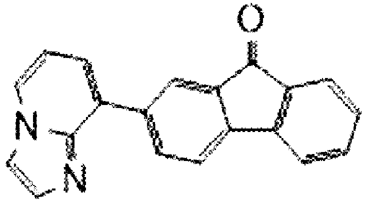

8.63(d, 1H), 8.00(d, 1H), 7.98~7.92(m, 3H), 7.86(S, 1H), 7.72~7.65(m, 3H), 7.43(t, 1H), 7.35(t, 1H), 7.01(t, 1H)
MS+:297.54

### [Example 1] Confirmation of STAT3 transcriptional activity inhibitory effect

For HEK293 cells, an immortalized human kidney cell line, transformed HEK293 cells, in which an STAT3 promoter and a luciferase reporter were stably expressed, were used. The HEK293 cells, in which the STAT3 promoter was stably expressed, were cultured in a cell incubator at 5% CO₂ and 37°C using an MEM (10% FBS) medium, and then an inhibitory effect on STAT3 transcriptional activity was confirmed by the following method.

First, HEK293 cells, in which the STAT3 promoter was stably expressed, were inoculated into a 96-well plate (white, clear bottom culture plate) at a density of 2X 104 cells/well, and then cultured in an incubator at 37°C and 5% CO₂ for 24 hours. Then, the compound synthesized according to the above synthesis example (1 µM) or BBI-608 (Napabucasin; comparative group 3 µM) and IL-6 (10 ng/mL) were treated together, and reacted in an incubator at 37°C and 5% CO₂ for 24 hours. Afterwards, the culture medium in the 96-well plate was removed, and the cells were lysed by treatment with Passive Lysis Buffer (Promega/Cat. Number E1941). Luciferase activity was tested using the Luciferase Assay System (Promega/Cat. Number E1501), and a luminescence value was measured using a microplate reader. A well not treated with IL-6 (10 ng/mL) without test substance treatment was used as a negative control, and a well treated with IL-6 (10 ng/mL) without test substance treatment was used as a positive control. A degree of STAT3 transcriptional activity inhibitory effect by the compound of the present invention was evaluated by comparing the luminescence values from each well.

**[Table 1]**

| STAT3 transcriptional activity inhibitory effect (compound concentration of 1 µM) | | | |
|---|---|---|---|
| % inhibition | | | |

| Example No. | 50% or less | 50-70% | 70% or more |
|---|---|---|---|
| BBI-608 (3 uM) | | ○ | |
| Compound 2 | ○ | | |
| Compound 3 | ○ | | |
| Compound 4 | | ○ | |
| Compound 5 | | ○ | |
| Compound 7 | | | ○ |
| Compound 8 | | | ○ |
| Compound 9 | | | ○ |
| Compound 10 | ○ | | |
| Compound 11 | ○ | | |
| Compound 12 | ○ | | |
| Compound 13 | | | ○ |
| Compound 14 | | | ○ |
| Compound 15 | | ○ | |
| Compound 16 | | ○ | |
| Compound 17 | ○ | | |
| Compound 18 | | ○ | |
| Compound 19 | ○ | | |
| Compound 20 | ○ | | |
| Compound 21 | | | ○ |
| Compound 22 | ○ | | |
| Compound 23 | | | ○ |

As a result, according to FIG. 1 and Table 1, it could be confirmed that compound 7, compound 8, compound 9, compound 13, compound 14, compound 21, and compound 23 exhibit an excellent STAT3 activity inhibitory effect even at a concentration less than that of BBI-608, in particular, as a comparative group, which is under an ongoing phase 3 clinical trial.

Although the present invention has been described with reference to limited embodiments in the present specification, various embodiments are possible within the spirit and scope of the present invention. Although not described, it will be said that even equivalent means are also combined with the present invention. Thus, the true technical protection scope of the present invention should be determined by the following claims.

## Claims

1. A tricyclic derivative compound represented by formula 1 below or a pharmaceutically acceptable salt thereof: 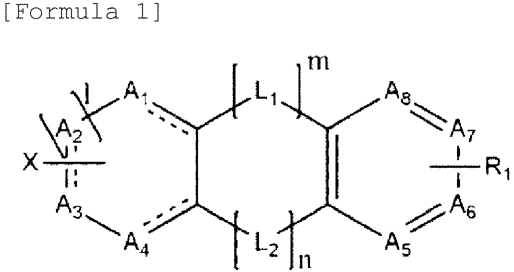 wherein in above formula 1, A₁ to A₈ are the same as or different from each other and are CH or N, of L₁ and L₂ are the same as or different from each other and are any one of CH₂, CO, NH, O, S, SO, SO₂, NR₂, and CHR₂, l and n are the same as or different from each other and are 0 or 1, m is 1 or 2, X is any one of H, halogen, (C1-C4)alkyl, and (C1-C4)alkoxy, R₁ is 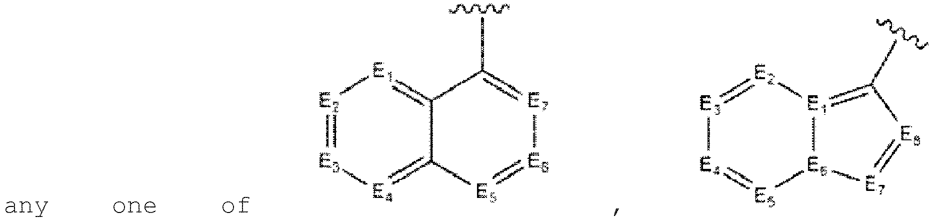 and 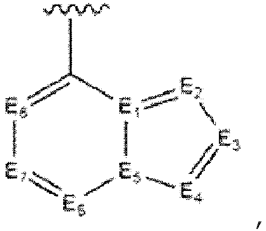 E₁ to E₈ are the same as or different from each other and are CH or N, and R₂ is selected from the group consisting of H, a hydroxy group, an ether group, halogen, a carbonyl group, a nitro group, a naphthyl group, a cyano group, an amino group, an amidino group, hydrazine, hydrazone, a carboxyl group, a sulfonic acid group, a phosphoric acid group, (C1-C12)alkyl, (C1-C12)alkenyl, (C1-C12)alkynyl, (C6-C10)aryl, and (C7-C10)arylalkyl.

2. The tricyclic derivative compound or the pharmaceutically acceptable salt thereof of claim 1, wherein above formula 1 is formula 1-1 below: 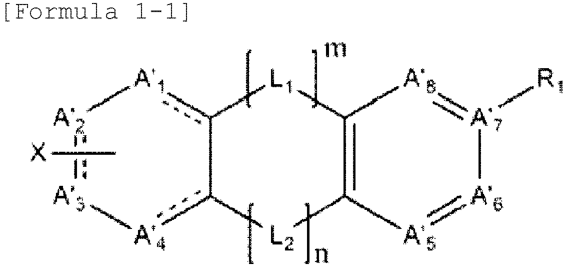 wherein in above formula 1-1, A'₁ to A'₈ are the same as or different from each other and are CH or N, L₁ and L₂ are the same as or different from each other and are any one of CH₂, CO, NH, O, S, and SO, n is 0 or 1, m is 1 or 2, and X is H or halogen; and R₁ is any one of 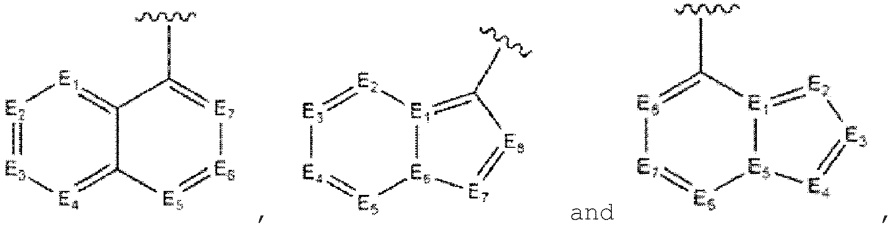 and E₁ to E₈ are the same as or different from each other and are CH or N.

3. The tricyclic derivative compound or the pharmaceutically acceptable salt thereof of claim 2, wherein in above formula 1-1, A'₁, A'₂, and A'₄ to A'₈ are CH, A'₃ is CH or N, L₁ and L₂ are the same as or different from each other and are any one of CO, NH, O and S, and X is H or halogen.

4. The tricyclic derivative compound or the pharmaceutically acceptable salt thereof of claim 2, wherein the compound is selected from the group consisting of 3-(isoquinolin-4-yl)-9H-fluoren-9-one, 2-bromo-7-(isoquinolin-4-yl)phenanthren-9,10-dione, 2-(isoquinolin-4-yl)-9H-xanthen-9-one, 2-(isoquinolin-4-yl)-9H-thioxanthen-9-one, 2-(pyrido[3,4-b]pyrazin-8-yl)-9H-fluoren-9-one, 2-(quinazolin-4-yl)-9H-fluoren-9-one, 2-(1,6-naphthyridin-8-yl)-9H-fluoren-9-one, 2-(1,6-naphthyridin-5-yl)-9H-fluoren-9-one, 2-(pyrazolo[1,5-a]pyridin-3-yl)-9H-fluoren-9-one, 2-(imidazo[1,2-a]pyridin-3-yl)-9H-fluoren-9-one, 4-(dibenzo[b,d]thiophen-3-yl)isoquinoline, 4-(dibenzo[b,d]furan-3-yl)isoquinoline, 7-(isoquinolin-4-yl)-5H-pyrido[4,3-b]indole, 2-(isoquinolin-4-yl)-9H-fluoren-9-one, 2-(isoquinolin-4-yl)-9H-carbazole, 7-(isoquinolin-4-yl)-2,3,4,9-tetrahydro-1H-carbazole, 2-(isoquinolin-8-yl)-9H-fluoren-9-one, 2-(isoquinolin-5-yl)-9H-fluoren-9-one, 2-(imidazo[1,2-a]pyridin-5-yl)-9H-fluoren-9-one, 2-([1,2,4]triazolo[4,3-a]pyridin-8-yl)-9H-fluoren-9-one, 2-(isoquinolin-1-yl)-9H-fluoren-9-one, and 2-(imidazo[1,2-a]pyridin-8-yl)-9H-fluoren-9-one.

5. The tricyclic derivative compound or the pharmaceutically acceptable salt thereof of claim 1, wherein above formula 1 is formula 1-2 below: 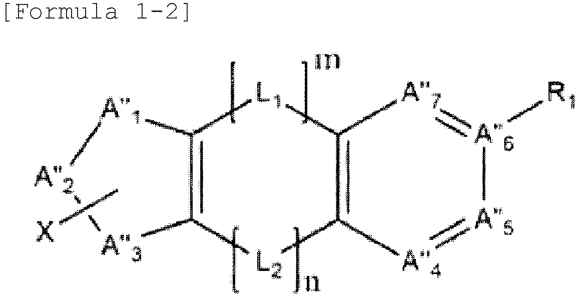 wherein in above formula 1-2, A"₁ to A"₇ are the same as or different from each other and are CH or N, L₁ and L₂ are the same as or different from each other and are any one of CH₂, CO, NH, O, S, and SO, n is 0 or 1, m is 1 or 2, and X is H or halogen; and R₁ is any one of 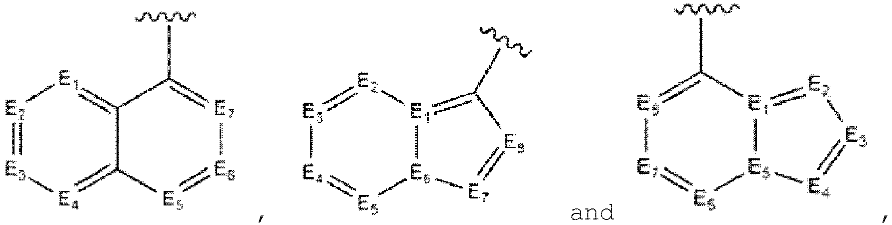 and E₁ to E₈ are the same as or different from each other and are CH or N.

6. The tricyclic derivative compound or the pharmaceutically acceptable salt thereof of claim 5, wherein in above formula 1-2, A₁ to A₇ are CH, n is 0, m is 1, L₁ is CH₂ or NH, and X is H.

7. The tricyclic derivative compound or the pharmaceutically acceptable salt thereof of claim 5, wherein the compound is 6-(isoquinolin-4-yl)-1,2,3,4-tetrahydrocyclopenta[b]indole.

8. A pharmaceutical composition for preventing or treating STAT3-associated diseases, comprising the compound of claim 1 or the pharmaceutically acceptable salt thereof as an active ingredient.

9. The pharmaceutical composition for preventing or treating STAT3-associated diseases of claim 8, wherein the STAT3-associated diseases are any one of cancer, osteoporosis, atopy, viral inflammatory disease, diabetic retinopathy, diabetes, hemophilic arthropathy, atherosclerosis, keloid, wound granulation, vascular adhesion, autoimmune disease, restenosis, intestinal tract adhesion, cat scratch disease, ulcer, cirrhosis, diabetic nephropathy, malignant nephrosclerosis, thrombotic microangiopathy, organ transplant rejection, glomerulopathy, neurodegenerative disease, and inflammatory disease.

10. A health functional food composition for preventing or ameliorating STAT3-associated diseases, comprising the compound of claim 1 or the pharmaceutically acceptable salt thereof as an active ingredient.

11. The health functional food composition for preventing or ameliorating STAT3-associated diseases of claim 10, wherein the STAT3-associated diseases are any one of cancer, osteoporosis, atopy, viral inflammatory disease, diabetic retinopathy, diabetes, hemophilic arthropathy, atherosclerosis, keloid, wound granulation, vascular adhesion, autoimmune disease, restenosis, intestinal tract adhesion, cat scratch disease, ulcer, cirrhosis, diabetic nephropathy, malignant nephrosclerosis, thrombotic microangiopathy, organ transplant rejection, glomerulopathy, neurodegenerative disease, and inflammatory disease.

12. A reagent composition for inhibiting STAT3 activity, comprising the compound of claim 1 or the pharmaceutically acceptable salt thereof as an active ingredient.
